# EUROPEAN PATENT APPLICATION

(11) **EP 4 474 481 A1**
(43) Date of publication of application: **11.12.2024**
(21) Application number: 23775241.5
(22) Date of filing: 20.03.2023
(51) Int. Cl.: C12P 3/00

(54) **METHOD FOR RECOVERING PHOSPHORIC ACID FROM FERMENTATION BROTH OR FERMENTATION WASTE LIQUID AND REUSING SAME**

(30) Priority: 24.03.2022 KR 20220036878
(71) Applicant: CJ CheilJedang Corporation, Seoul 04560 (KR)
(72) Inventor: LEE, Kang Hoon, Seoul 04560 (KR); LEE, Seung-je, Seoul 04560 (KR); KIM, Jun-Woo, Seoul 04560 (KR); KIM, Jaeik, Seoul 04560 (KR); LEE, In Sung, Seoul 04560 (KR); JUNG, Jun Young, Seoul 04560 (KR)
(74) Representative: Meissner Bolte Partnerschaft mbB
(86) International application number: PCT/KR2023/003652
(87) International publication number: WO 2023/182744

(57) **Abstract**

Provided are a method of recovering phosphoric acid in the form of ammonium phosphate or a hydrate thereof from a fermentation broth or a waste liquid thereof, and/or a method of reusing the recovered phosphoric acid in fermentation.

## Description

### [Technical Field]

The present disclosure relates to a method of recovering phosphoric acid in the form of ammonium phosphate or a hydrate thereof from a fermentation broth or a waste liquid thereof, and/or a method of reusing the recovered phosphoric acid in fermentation.

### [Background Art]

In some cases, phosphoric acid is essentially required to produce a target substance by fermentation. For example, International Patent Application Publication No. WO 2014/182125 discloses that a phosphoric acid source may be provided for fermentation of O-phosphoserine, which is a precursor used to produce L-cysteine. Further, International Patent Application Publication No. WO 2014/064244 discloses a method of producing methionine from O-phosphohomoserine by using a sulfide and a converting enzyme. Based thereon, it can be confirmed that a phosphate is essential as a phosphorus source to produce O-phosphohomoserine, which is a precursor of methionine, by fermentation. Therefore, there is a need to develop a method of recovering residual phosphoric acid from a fermentation broth or waste liquid thereof including a large amount of a phosphate and reusing the recovered phosphoric acid as a phosphorus source.

Existing processes of recovering phosphoric acid are generally performed by isolating phosphoric acid from phosphoric acid-containing rock materials using an organic solvent extraction process, and then recovering phosphoric acid through a neutralization process with a calcium salt, an ammonium salt, a potassium salt, a sodium salt, or the like (US3375068, US3466141A, US4543239, EP0087323A1, US7687046, US8658117). However, the organic solvent extraction process is economically inefficient, because processing costs increase due to use of the solvent, and the process is complicated due to an additional procedure of recovering the solvent. Further, an available organic solvent may act as a toxin in a fermentation process even in a small amount, and thus it is difficult to apply the organic solvent to the fermentation process.

Meanwhile, Korean Patent Publication No. 10-2025867 discloses a method of recovering phosphoric acid from a fermentation broth or a waste liquid thereof, and a process of reusing the recovered phosphoric acid in fermentation. However, phosphoric acid is recovered only in the form of disodium hydrogen phosphate. There is no mention of a method of recovering phosphoric acid in the form of ammonium phosphate or a hydrate thereof.

In view of this background, the present inventors have endeavored to develop a method of recovering phosphoric acid in the form of ammonium phosphate or a hydrate thereof from a fermentation broth or a fermentation waste liquid, and as a result, they found that phosphoric acid may be recovered in the form of ammonium phosphate or a hydrate thereof from a fermentation broth or fermentation waste liquid without using an organic solvent, and when the recovered phosphoric acid is reused in fermentation, a fermentation product may be obtained in an amount similar to that obtained using pure phosphoric acid, thereby completing the present disclosure.

### [Disclosure]

### [Technical Problem]

The present inventors develop a method of recovering phosphoric acid in the form of ammonium phosphate or a hydrate thereof from a fermentation broth or a fermentation waste liquid without using an organic solvent, and/or a method of reusing the recovered phosphoric acid in fermentation, thereby completing the present disclosure.

### [Technical Solution]

An object of the present disclosure is to provide a method of recovering phosphoric acid in the form of ammonium phosphate or a hydrate thereof from a phosphoric acid-containing fermentation broth or fermentation waste liquid.

Another object of the present disclosure is to provide a method of reusing recovered phosphoric acid by separating the phosphoric acid in the form of ammonium phosphate or a hydrate thereof from a phosphoric acid-containing fermentation broth or fermentation waste liquid.

### [Advantageous Effects]

When phosphoric acid is recovered from a fermentation process, not from rock materials, a method of recovering phosphoric acid in the form of ammonium phosphate or a hydrate thereof by a method other than an organic solvent extraction method and reusing the same in a fermentation process has been identified for the first time in the present disclosure.

According to the method of recovering phosphoric acid in the form of ammonium phosphate or a hydrate thereof from a fermentation medium and reusing the same in the present disclosure, phosphoric acid may be recovered in the form of ammonium phosphate or a hydrate thereof from a fermentation broth or a fermentation waste liquid without using an organic solvent and may be reused in a fermentation process. Therefore, problems caused by using an organic solvent, such as acting as a toxin in a fermentation process, additional costs incurred for using the solvent, and complicating a process due to addition of a solvent-recovering process, may be overcome, and thus, the method may be very usefully used in a fermentation process.

Further, unlike the existing method of recovering phosphoric acid in the form of disodium hydrogen phosphate (Korean Patent Publication No. 10-2025867), the method of recovering phosphoric acid in the form of ammonium phosphate or a hydrate thereof from a fermentation medium and reusing the same according to the present disclosure may recover phosphoric acid in the form of ammonium phosphate or a hydrate thereof to decrease the content of sodium ions and to increase ammonium ions in the recovered phosphoric acid. Accordingly, when the recovered phosphoric acid having a relatively high ratio of ammonia is reused in a fermentation process of O-phosphoserine (OPS), which has a long fermentation time, there are advantages in that the productivity increases and the culture time is reduced, and therefore, the method may also be very usefully used in a fermentation process with a long fermentation time.

Further, since the method of recovering phosphoric acid in the form of ammonium phosphate or a hydrate thereof from a fermentation medium and reusing the same according to the present disclosure uses ammonia gas or an ammonia solution in the pH-adjusting step, the phosphoric acid recovery rate is increased, as compared to the existing method (Korean Patent Publication No. 10-2025867) of recovering phosphoric acid, in which the phosphoric acid recovery rate is reduced due to dilution by using a sodium hydroxide solution in the pH-adjusting step.

Further, since the method of recovering phosphoric acid in the form of ammonium phosphate or a hydrate thereof from a fermentation medium and reusing the same according to the present disclosure may increase the ammonia ratio in the process solution by using ammonia, sodium ions may be decreased and ammonium ions may be increased in the recovered phosphoric acid waste liquid, and therefore, it may be usefully used as a high-value fertilizer.

### [Description of Drawings]

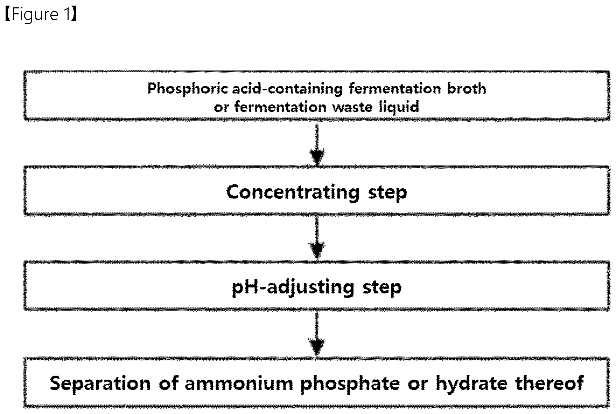

FIG. 1 shows a schematic illustration of a process of recovering ammonium phosphate or a hydrate thereof from a phosphoric acid-containing fermentation broth or fermentation waste liquid;

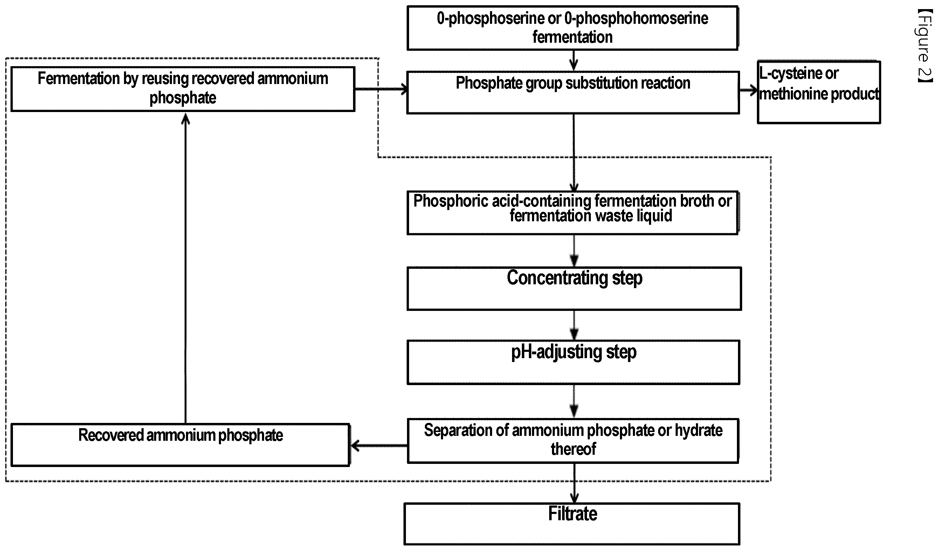

FIG. 2 shows a schematic illustration of a process of recovering ammonium phosphate or a hydrate thereof from a fermentation broth which was produced by an enzymatic conversion reaction from an O-phosphoserine (OPS) or O-phosphohomoserine (OPHS) fermentation broth, or from a fermentation waste liquid which was obtained by removing cysteine, cysteine derivatives, methionine, or methionine derivatives from the fermentation broth; and reusing the corresponding recovered phosphoric acid in the fermentation of O-phosphoserine or O-phosphohomoserine; and

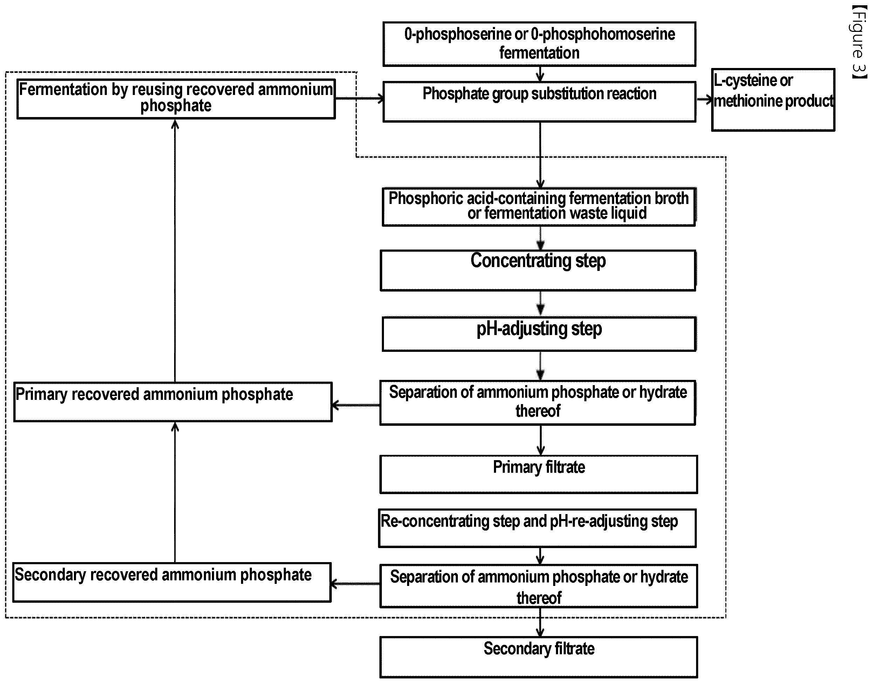

FIG. 3 shows a schematic illustration of a process of recovering ammonium phosphate or a hydrate thereof via a two-step process from a fermentation broth which was produced by an enzymatic conversion reaction from an O-phosphoserine or O-phosphohomoserine fermentation broth, or from a fermentation waste liquid which was obtained by removing cysteine, cysteine derivatives, methionine, or methionine derivatives from the fermentation broth; and reusing the recovered phosphoric acid in the fermentation of O-phosphoserine or O-phosphohomoserine.

### [Best Mode]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

To achieve the above objects, one aspect of the present disclosure provides a method of recovering phosphoric acid from a fermentation broth or a fermentation waste liquid, the method including the steps of:
(a) concentrating a phosphoric acid-containing fermentation broth or fermentation waste liquid (concentrating step);
(b) adjusting pH of the concentrated solution at 8 to 11 by adding ammonia gas or an ammonia solution to the concentrated solution (pH-adjusting step); and
(c) separating ammonium phosphate or a hydrate thereof from the pH-adjusted concentrated solution (crystallizing step).

Another aspect of the present disclosure provides a method of reusing phosphoric acid from a fermentation broth or fermentation waste liquid, the method including the steps of:
(a) concentrating a phosphoric acid-containing fermentation broth or fermentation waste liquid (concentrating step);
(b) adjusting pH of the concentrated solution at 8 to 11 by adding ammonia gas or an ammonia solution to the concentrated solution (pH-adjusting step);
(c) separating ammonium phosphate or a hydrate thereof from the pH-adjusted concentrated solution (crystallizing step); and
(d) adding the ammonium phosphate or the hydrate thereof to a medium.

Unlike the existing method of recovering phosphoric acid in the form of disodium hydrogen phosphate (Korean Patent Publication No. 10-2025867), the method of recovering phosphoric acid in the form of ammonium phosphate or a hydrate thereof from a fermentation medium and reusing the same according to the present disclosure may recover phosphoric acid in the form of ammonium phosphate or a hydrate thereof to decrease the content of sodium ions and to increase ammonium ions in the recovered phosphoric acid. Accordingly, when phosphoric acid recovered according to the method of the present disclosure is reused in a fermentation process with a long fermentation time, for example, a fermentation process of O-phosphoserine (OPS), etc., there are advantages in that the productivity is increased and the culture time is reduced, and therefore, the method may be very usefully used.

As used herein, the term "fermentation broth" comprehensively refers to a medium obtained by culturing a microorganism producing a fermentation product in a medium containing phosphoric acid, a culture containing the microorganism cultured together with the medium, or an enzyme conversion solution thereof, etc.

Specifically, the fermentation broth may include (1) a fermentation broth of a target substance containing phosphoric acid, a precursor thereof, or a derivative thereof, (2) a fermentation broth including a target substance or derivatives thereof, produced by a strain producing a precursor of the target substance, a substrate, and a converting enzyme; and phosphoric acid, or (3) a fermentation broth including a target substance or derivatives thereof, produced by adding a converting enzyme or a microorganism expressing the converting enzyme; and a substrate to a fermentation broth of a precursor of the target substance; and phosphoric acid, but is not limited thereto.

More specifically, the fermentation broth may include (i) O-phosphoserine, (ii) O-phosphohomoserine, or (iii) an amino acid or derivatives thereof, prepared by adding O-phosphoserine or O-phosphohomoserine, a converting enzyme or a microorganism expressing the same, and a sulfide, but is not limited thereto.

More specifically, the fermentation broth may be (i) an O-phosphoserine fermentation broth including phosphoric acid, (ii) an O-phosphohomoserine (OPHS) fermentation broth including phosphoric acid, or (iii) a fermentation broth including an amino acid or derivatives thereof, prepared by adding a converting enzyme or a microorganism expressing the same; and a sulfide to the fermentation broth; and phosphoric acid. Further, the (iii) fermentation broth may be specifically a fermentation broth including cysteine or derivatives thereof, prepared by adding O-phosphoserine sulfhydrylase (OPSS) or a microorganism expressing the same; and a sulfide to the O-phosphoserine fermentation broth; and phosphoric acid. Further, the fermentation broth (iii) may be a fermentation broth including methionine or derivatives thereof, prepared by adding an O-phospho-homoserine dependent (OPHS-dependent) methionine synthase or a microorganism expressing the same; and a sulfide to the O-phosphohomoserine fermentation broth; and phosphoric acid.

The type of the 'fermentation product' is not limited as long as the fermentation broth includes phosphoric acid, but may be specifically a target substance, a derivative or precursor thereof, which is produced by fermentation. Further, the target substance may be an amino acid, for example, cysteine or methionine, but is not limited thereto. Further, the derivative of the target substance may be a derivative of an amino acid, specifically, a derivative of cysteine or a derivative of methionine. More specifically, the derivative of cysteine may include one or more selected from the group consisting of N-acetyl-cysteine, cystine, a metal-cysteine complex (e.g., zinc-cysteine complex, manganese-cysteine complex, iron-cysteine complex, copper-cysteine complex, and magnesium-cysteine complex, etc.) and a cysteine salt (e.g., cysteine hydrochloride, etc.), but is not limited thereto. The derivative of methionine may include one or more selected from the group consisting of N-acetyl-methionine, a metal-methionine complex (e.g., zinc-methionine complex, manganese-methionine complex, iron-methionine complex, copper-methionine complex, and magnesium-methionine complex, etc.), and a methionine salt, etc., but is not limited thereto. Further, the precursor of the target substance may be a precursor of an amino acid, specifically, a precursor of cysteine or a precursor of methionine. More specifically, the precursor may be O-phosphoserine (OPS) or O-phosphohomoserine (OPHS), but is not limited thereto.

As used herein, the term "fermentation waste liquid" may be a liquid obtained by partially or entirely separating and removing the fermentation product from the fermentation broth, but is not limited thereto. Specifically, the fermentation waste liquid may be a liquid obtained by partially or entirely separating and removing an amino acid or derivatives or precursors thereof from a fermentation broth including the amino acid or derivatives or precursors thereof and phosphoric acid, but is not limited thereto. For example, the fermentation waste liquid may be a liquid obtained by partially or entirely separating and removing an amino acid or derivatives thereof from a fermentation broth including the amino acid and derivatives thereof and phosphoric acid or a liquid obtained by partially or entirely separating and removing an amino acid or derivatives thereof from a fermentation broth including the amino acid or derivatives thereof, produced by adding a substrate and a converting enzyme, or a microorganism producing the converting enzyme to a fermentation broth of a precursor of the amino acid, and phosphoric acid. More specifically, the fermentation waste liquid may be a liquid obtained by partially or entirely separating and removing cysteine or derivatives thereof from a fermentation broth including the cysteine or derivatives thereof prepared by adding O-phosphoserine sulfhydrylase (OPSS) or a microorganism expressing the same; and a sulfide to the O-phosphoserine fermentation broth; and phosphoric acid. Alternatively, the fermentation waste liquid may be a liquid obtained by partially or entirely separating and removing methionine or derivatives thereof from a fermentation broth including methionine or derivatives thereof, prepared by adding O-phospho-homoserine dependent methionine synthase (OPHS-dependent methionine synthase) or a microorganism expressing the same; and a sulfide to the O-phosphohomoserine fermentation broth, and phosphoric acid, but is not limited thereto.

As used herein, the term "concentrating" may be performed by any known method without limitation as long as the concentration of phosphate ions included in the fermentation broth or fermentation waste liquid is increased by the method. Specifically, the concentrating may be performed by evaporating, heating, depressurizing, ventilating, freezing, or the like, but is not limited thereto.

In the present disclosure, the concentrating is performed to increase a recovery rate of phosphoric acid, and the fermentation broth or fermentation waste liquid may be concentrated in the concentrating step such that the concentration of phosphate ions in the pH-adjusted concentrated solution may be specifically 60 g/L or more or 70 g/L or more, and more specifically, 60 g/L to 300 g/L or 70 g/L to 250 g/L, but is not limited thereto.

Further, in the present disclosure, before step (a) (concentrating step), the step of adjusting the pH of the fermentation broth or fermentation waste liquid may be further included. Specifically, the pH of the fermentation broth or fermentation waste liquid may be adjusted at 7 or more, 7.5 or more, 8 or more, 8.5 or more, or 9 or more, more specifically, 8 to 11.

In the present disclosure, step (b) (pH-adjusting step) is a step of adjusting the pH of the concentrated solution before crystallizing the phosphate. The pH of the concentrated solution obtained in the concentrating step is not limited as long as the phosphate is crystallized at the pH, but the pH of the concentrated solution may be adjusted from neutral to alkaline, specifically, at 7 or more, 7.5 or more, 8 or more, 8.5 or more, or 9 or more, more specifically, 8 to 11.

In addition, in the present disclosure, the pH-adjusting step may be performed by adding an alkaline substance to the concentrated solution, specifically, by adding ammonia gas (NH3 (g)) or an ammonia solution (NH4OH (liq)).

In particular, in the present disclosure, when ammonia gas or ammonia solution is used in the pH-adjusting step, the recovery rate of phosphoric acid is increased, as compared to the existing method of recovering phosphoric acid, in which a sodium hydroxide solution is used in the pH-adjusting step (Korean Patent Publication No. 10-2025867).

In the present disclosure, step (c) (crystallizing step) is a step of separating ammonium phosphate or a hydrate thereof from the pH-adjusted concentrated solution, which is obtained in the pH-adjusting step. In the present disclosure, the separating of ammonium phosphate or a hydrate thereof is performed by generating crystals of ammonium phosphate from the pH-adjusted concentrated solution and separating the generated crystals from a mother liquor without using an organic solvent.

For crystallization of ammonium phosphate, temperature may be controlled and/or crystal nucleus may be added thereto, but is not limited thereto. Specifically, for crystallization of ammonium phosphate, a step of cooling the pH-adjusted concentrated solution may be further included between step (b) (pH-adjusting step) and step (c) (crystallizing step), or in step (c) (crystallizing step). More specifically, the pH-adjusted concentrated solution may be left to stand at room temperature, the pH-adjusted concentrated solution may be cooled before the crystallizing step, or the pH-adjusted concentrated solution may be cooled in the crystallizing step, but is not limited thereto. Meanwhile, although the recovery rate of phosphoric acid may be affected by the cooling temperature, the cooling temperature is not limited as long as crystals of ammonium phosphate are generated. Specifically, the cooling temperature may be 50°C or lower, specifically, 0°C to 30°C, more specifically, 10°C to 20°C, but is not limited thereto.

Further, specifically, for crystallization of ammonium phosphate, a step of adding the crystals of ammonium phosphate or hydrate thereof as crystal nuclei may be further included between step (b) (pH-adjusting step) and step (c) (crystallizing step) or in step (c) (crystallizing step), but is not limited thereto.

As used herein, the term 'phosphate', which is a salt of phosphoric acid or a hydrate thereof, may refer to ammonium phosphate or a hydrate thereof, but the type of the salt is not limited thereto as long as phosphoric acid may be recovered according to the method of the present disclosure. Specifically, the ammonium phosphate may include one or more selected from triammonium phosphate, diammonium hydrogen phosphate (ammonium phosphate dibasic), and ammonium dihydrogen phosphate, more specifically, diammonium hydrogen phosphate, but is not limited thereto. The number of water molecules bound to the hydrate of ammonium phosphate of the present disclosure is not limited as long as the hydrate of ammonium phosphate is crystallized, but may be, specifically, 1, 2, 3, 4, 5, 6, 7, 8, 9, 10, 11, 12, or 13 or more. In addition, the hydrate of ammonium phosphate of the present disclosure may be specifically diammonium hydrogen phosphate heptahydrate or diammonium hydrogen phosphate dodecahydrate, but is not limited thereto.

In the present disclosure, phosphoric acid and phosphate may be used interchangeably.

In the present disclosure, any method enabling solid-liquid separation may be used to separate the generated phosphate crystals without limitation, and specifically, a centrifuge, a filter press, a compression filter, a rotary vacuum filter, a membrane separator, or the like may be used, but is not limited thereto.

In the present disclosure, the mother liquor refers to a liquid obtained by separating and removing the generated phosphate crystals from the pH-adjusted concentrated solution and may include uncrystallized phosphate ions, but is not limited thereto.

In order to increase the recovery rate of phosphoric acid, one embodiment of the method of the present disclosure may further include a second crystallizing step of re-crystallizing the phosphate (ammonium phosphate or hydrate thereof in step (c)) from the mother liquor. In the present disclosure, a high recovery rate of phosphoric acid may be obtained by adding phosphate crystals (secondary recovered phosphate) obtained in the second crystallizing step to phosphate crystals (primary recovered phosphate) obtained in the previous step (c) (crystallizing step). Specifically, the second crystallizing step may include (i) the step of concentrating a mother liquor (re-concentrating step), (ii) the step of adjusting pH of the mother liquor (pH re-adjusting step), and (iii) the step of crystallizing the phosphate in the pH-adjusted mother liquor and separating the crystalized phosphate therefrom (re-crystallizing step). The steps (i) to (iii) may be performed in the same manner as the concentrating step, the pH-adjusting step, and the crystallizing step described above. Specifically, the pH of the mother liquor may be adjusted at 7 or more, 7.5 or more, 8 or more, 8.5 or more, or 9 or more, more specifically, 8 to 11.

In addition, specifically, the second crystallizing step may further include the step of adding phosphate crystals, as crystal nuclei, to the mother liquor, for example, the step of adding crystals of ammonium phosphate or hydrate thereof, as crystal nuclei, to the mother liquor, between step (ii) (pH re-adjusting step) and step (iii) (re-crystallizing step) or in step (iii) (re-crystallizing step), but is not limited thereto.

In addition, in the present disclosure, the crystallizing step may be performed more than twice to increase the recovery rate of phosphoric acid.

The method of the present disclosure may further include the step of adding alcohol in step (b) (pH re-adjusting step); or between step (b) (pH re-adjusting step) and step (c) (crystallizing step). Due to the low solubility of phosphate in alcohol, the alcohol, when mixed with a phosphate solution, reduces the solubility of the phosphate in the solution, and thus plays a role in increasing the recovery rate of phosphoric acid. The alcohol is not limited as long as the recovery rate of phosphoric acid is increased, but may be specifically methanol, ethanol, propanol, butanol, or a combination thereof.

One embodiment of the method of the present disclosure may further include the step of using (re-using) the ammonium phosphate or hydrate thereof of step (c) as a source of phosphoric acid in a fermentation medium. In the present disclosure, the re-using step may be a step of using the phosphate crystals recovered from the crystallizing step as a source of phosphoric acid and/or phosphorus in various fermentation media requiring the source of phosphoric acid and/or phosphorus. The fermentation medium of the present disclosure is not limited as long as the medium is used in fermentation requiring a source of phosphoric acid and/or phosphorus with respect to the objects of the present disclosure, but the fermentation medium may be specifically a fermentation medium used in the production of O-phosphoserine or O-phosphohomoserine.

Another aspect of the present disclosure provides a method of recovering phosphoric acid from a fermentation broth or a fermentation waste liquid, the method including the steps of:
(a) producing O-phosphoserine (OPS) using a microorganism in a fermentation medium containing phosphoric acid (OPS fermentation step);
(b) preparing a fermentation broth of cysteine or a derivative thereof by reacting O-phosphoserine produced in step (a) with a sulfide in the presence of O-phosphoserine sulfhydrylase (OPSS) or a microorganism expressing the OPSS (converting step);
(c) concentrating the fermentation broth; or a fermentation waste liquid obtained by removing cysteine or derivatives thereof from the fermentation broth (concentrating step);
(d) adjusting pH of the concentrated solution at 8 to 11 by adding ammonia gas or an ammonia solution to the concentrated solution (pH-adjusting step); and
(e) separating ammonium phosphate or a hydrate thereof from the pH-adjusted concentrated solution (crystallizing step).

Still another aspect of the present disclosure provides a method of reusing phosphoric acid from a fermentation broth or a fermentation waste liquid, the method including the steps of:
(a) producing O-phosphoserine (OPS) using a microorganism in a fermentation medium containing phosphoric acid (OPS fermentation step);
(b) preparing a fermentation broth of cysteine or a derivative thereof by reacting O-phosphoserine produced in step (a) with a sulfide in the presence of O-phosphoserine sulfhydrylase (OPSS) or a microorganism expressing the OPSS (converting step);
(c) concentrating the fermentation broth; or a fermentation waste liquid obtained by removing cysteine or derivatives thereof from the fermentation broth (concentrating step);
(d) adjusting pH of the concentrated solution at 8 to 11 by adding ammonia gas or an ammonia solution to the concentrated solution (pH-adjusting step);
(e) separating ammonium phosphate or a hydrate thereof from the pH-adjusted concentrated solution (crystallizing step); and
(f) adding the ammonium phosphate or the hydrate thereof to a medium.

In the present disclosure, step (a) (OPS fermentation step) is a step of producing O-phosphoserine by using a fermentation medium containing phosphoric acid and a microorganism. O-phosphoserine is an ester of serine and phosphoric acid, and phosphoric acid is required to produce O-phosphoserine. Further, the microorganism may be any known microorganism capable of producing O-phosphoserine, and nonlimiting examples thereof may include microorganisms having enhanced activity of exporting O-phosphoserine (International Patent Application Publication Nos. WO 2014/182125 and WO 2014/182119). In addition, examples of microorganisms having high O-phosphoserine-producing ability may include a microorganism having reduced activity of endogenous phosphoserine phosphatase (SerB) and/or enhanced activity of phosphoglycerate dehydrogenase (SerA) and/or phosphoserine aminotransferase (SerC) (International Patent Application Publication No. WO 2012/053794), but are not limited thereto.

In the present disclosure, step (b) (converting step) is a step of converting O-phosphoserine, obtained in the OPS fermentation step, into cysteine or derivatives thereof by reacting O-phosphoserine with a sulfide under the catalytic action of O-phosphoserine sulfhydrylase enzyme.

Further, in the present disclosure, the conversion reaction may also be performed by using O-phosphoserine sulfhydrylase enzyme as well as a microorganism expressing the O-phosphoserine sulfhydrylase. The enzyme and the microorganism expressing the enzyme may be obtained by any means and method known to those skilled in the art. Specifically, the O-phosphoserine sulfhydrylase enzyme may be any known enzyme disclosed in International Patent Application Publication Nos. WO 2013/089478, WO 2012/053794, WO 2012/053777, etc., but is not limited thereto.

Further, in the present disclosure, the sulfide is not limited as long as the sulfide reacts with O-phosphoserine under the catalytic action of O-phosphoserine sulfhydrylase enzyme, but may be one or more selected from the group consisting of CH₃SH, Na₂S, NaSH, (NH₄)₂S, H₂S, and Na₂S₂O₃.

In the present disclosure, the steps (c) to (e) are the same as the concentrating step, the pH-adjusting step, and the crystallizing step described above.

One embodiment of the method of the present disclosure may further include step (reusing step) of using the ammonium phosphate or hydrate thereof of step (e) as a source of phosphoric acid in a fermentation medium. In the present disclosure, the reusing step is a step of using the phosphate crystals recovered from the crystallizing step, as a source of phosphoric acid and/or phosphorus, in a fermentation medium requiring the source of phosphoric acid and/or phosphorus. Specifically, the fermentation medium may be any fermentation medium used to produce O-phosphoserine, but is not limited thereto.

Still another aspect of the present disclosure provides a method of recovering phosphoric acid from a fermentation broth or a fermentation waste liquid, the method including the steps of:
(a) producing O-phosphohomoserine (OPHS) using a microorganism in a fermentation medium containing phosphoric acid (OPHS fermentation step);
(b) preparing a fermentation broth of methionine or a derivative thereof by reacting O-phosphohomoserine produced in step (a) with a sulfide in the presence of O-phosphohomoserine-dependent methionine synthase or a microorganism expressing the O-phosphohomoserine-dependent methionine synthase (converting step);
(c) concentrating the fermentation broth; or a fermentation waste liquid obtained by removing methionine or a derivative thereof from the fermentation broth (concentrating step);
(d) adjusting pH of the concentrated solution at 8 to 11 by adding ammonia gas or an ammonia solution to the concentrated solution (pH-adjusting step); and
(e) separating ammonium phosphate or a hydrate thereof from a mother liquor in the pH-adjusted concentrated solution (crystallizing step).

Still another aspect of the present disclosure provides a method of reusing phosphoric acid from a fermentation broth or a fermentation waste liquid, the method including the steps of:
(a) producing O-phosphohomoserine (OPHS) using a microorganism in a fermentation medium containing phosphoric acid (OPHS fermentation step);
(b) preparing a fermentation broth of methionine or a derivative thereof by reacting O-phosphohomoserine produced in step (a) with a sulfide in the presence of O-phosphohomoserine-dependent methionine synthase or a microorganism expressing the O-phosphohomoserine-dependent methionine synthase (converting step);
(c) concentrating the fermentation broth; or a fermentation waste liquid obtained by removing methionine or a derivative thereof from the fermentation broth (concentrating step);
(d) adjusting pH of the concentrated solution at 8 to 11 by adding ammonia gas or an ammonia solution to the concentrated solution (pH-adjusting step);
(e) separating ammonium phosphate or a hydrate thereof from the pH-adjusted concentrated solution (crystallizing step); and
(f) adding the ammonium phosphate or the hydrate thereof to a medium.

In the present disclosure, step (a) (OPHS fermentation step) is a step of producing O-phosphohomoserine by using a fermentation medium containing phosphoric acid and a microorganism. O-phosphohomoserine is an ester of threonine and phosphoric acid, and phosphoric acid is required to produce O-phosphohomoserine. Further, the microorganism may be any known microorganism capable of producing O-phosphohomoserine.

In the present disclosure, step (b) (converting step) is a step of converting O-phosphohomoserine into methionine or derivatives thereof by reacting O-phosphohomoserine obtained from the OPHS fermentation step with a sulfide under the catalytic action of the OPHS-dependent methionine synthase.

Further, in the present disclosure, the conversion reaction may also be performed by using an OPHS-dependent methionine synthase enzyme as well as a microorganism expressing the OPHS-dependent methionine synthase. Specifically, the enzyme and the microorganism expressing the enzyme may be obtained by any means and method known to those skilled in the art, for example, by a method disclosed in International Patent Application Publication No. WO 2014/064244, but is not limited thereto.

Further, in the present disclosure, the sulfide is not limited as long as the sulfide reacts with O-phosphohomoserine under the catalytic action of OPHS-dependent methionine synthase, but may include one or more selected from the group consisting of CH₃SH, Na₂S, NaSH, (NH₄)₂S, H₂S, and Na₂S₂O₃.

In the present disclosure, the steps (c) to (e) are the same as the concentrating step, the pH-adjusting step, and the crystallizing step described above.

One embodiment of the method of the present disclosure may further include the step (reusing step) of using the ammonium phosphate or the hydrate thereof of step (e) as a source of phosphoric acid in a fermentation medium. In the present disclosure, the reusing step is a step of using the phosphate crystals recovered from the crystallizing step, as a source of phosphoric acid and/or phosphorus, in a fermentation medium requiring the source of phosphoric acid and/or phosphorus. Specifically, the fermentation medium may be any fermentation medium used to produce O-phosphohomoserine, but is not limited thereto.

Still another aspect of the present disclosure provides a method of recovering phosphoric acid from a fermentation waste liquid.

Specifically, the method of the present disclosure includes the steps of:
(a) concentrating a fermentation waste liquid containing phosphoric acid (concentrating step);
(b) adjusting pH of the concentrated solution at 8 to 11 by adding ammonia gas or an ammonia solution to the concentrated solution (pH-adjusting step); and
(c) separating ammonium phosphate or the hydrate thereof from the pH-adjusted concentrated solution (crystallizing step).

In the present disclosure, the fermentation waste liquid is as described above, and may refer to a waste liquid of enzymatic conversion.

In the present disclosure, the steps (a) to (c) are the same as the concentrating step, the pH-adjusting step, and the crystallizing step described above.

The method of recovering phosphoric acid in the form of ammonium phosphate or a hydrate thereof from a fermentation medium and reusing the same according to the present disclosure may increase the ammonia ratio in the process solution by using ammonia, and thus sodium ions may be decreased and ammonium ions may be increased in the recovered phosphoric acid waste liquid, and accordingly, it may be usefully used as a high-value fertilizer.

### [Mode for Invention]

Hereinafter, the present disclosure will be described in more detail with reference to the following exemplary embodiments. However, the following exemplary embodiments are for illustrative purposes only, and the scope of the present disclosure is not intended to be limited by the following exemplary embodiments.

### Comparative Example 1. Method of recovering phosphoric acid in the form of disodium hydrogen phosphate using O-phosphoserine fermentation waste liquid

After an O-phosphoserine fermentation broth was obtained by culturing a microorganism capable of producing O-phosphoserine (OPS) in a fermentation medium including phosphoric acid, the fermentation broth was reacted with a sulfide using O-phosphoserine sulfhydrylase (OPSS) to obtain a fermentation broth including cysteine or cystine (International Patent Application Publication No. WO 2012/053794). Cysteine or cystine was crystallized from the fermentation broth and separated therefrom by solid-liquid separation to obtain a fermentation waste liquid.

The O-phosphoserine fermentation waste liquid had an ion composition (g/L) of 28.7 of sodium ions, 3.9 of ammonium ions, 40.3 of phosphate ions, and 52.1 of sulfate ions. Concentration was performed by evaporating 1747 ml of water from 2112 ml of the initial waste liquid. The concentration of phosphate ions of this concentrated solution was 210.6 g/L.

Subsequently, a 50% (w/w) aqueous sodium hydroxide solution (47.0 ml) was added thereto until pH reached 9.00. The concentration of phosphate ions of this solution was 179.1 g/L. Cooling was performed to 150°C to obtain disodium hydrogen phosphate heptahydrate. The slurry was filtered using a centrifugal basket filter and washed with pure water.

### Example 1. Method of recovering phosphoric acid in the form of ammonium phosphate using O-phosphoserine fermentation waste liquid

An O-phosphoserine fermentation waste liquid and a concentrated solution, each having the same composition, were obtained in the same manner as in Comparative Example 1.

Subsequently, 99% (vol%) ammonia gas (16.4 g), instead of the aqueous sodium hydroxide solution, was added to the concentrated solution having 210.6 g/L of the phosphate ion concentration, which was obtained in the same manner as in Comparative Example 1, until pH reached 9.00. The concentration of phosphate ions of this solution was 198.2 g/L. Cooling was performed to 150°C to obtain diammonium hydrogen phosphate heptahydrate. The slurry was filtered using a centrifugal basket filter and washed with pure water.

### Experimental Example 1. Comparison of main composition of recovered phosphoric acid using O-phosphoserine fermentation waste liquid

The main composition contents (%) of phosphoric acid recovered in Comparative Example 1 and Example 1 are shown in Table 1 below.

**[Table 1]**

| | Comparative Example 1 | Experimental Example 1 |
|---|---|---|
| Sodium content (%) | 18.3 | 11.0 |
| Ammonium content (%) | 2.1 | 7.1 |
| Phosphate content (%) | 42.4 | 42.8 |
| Sulfate content (%) | 2.2 | 4.1 |

As a result, as shown in Table 1, the main composition contents (%) of diammonium hydrogen phosphate recovered by the method of Example 1 were 11.0 of sodium, 7.1 of ammonium, 42.8 of phosphate, and 4.1 of sulfate. In addition, the main composition contents (%) of disodium hydrogen phosphate recovered by the method of Comparative Example 1 were 18.3 of sodium, 2.1 of ammonium, 42.4 of phosphate, and 2.2 of sulfate.

In other words, the substance recovered by the method of Example 1 had the phosphate content similar to that of the substance recovered by the method of Comparative Example 1, but its ammonium content showed about 5% increase and its sodium content showed about 7.3% decrease.

These results show that the method of recovering phosphoric acid in the form of ammonium phosphate or a hydrate thereof from a fermentation medium and reusing the same according to the present disclosure may decrease the sodium ion content and may increase the ammonium ions in the recovered phosphoric acid by recovering phosphoric acid in the form of ammonium phosphate or a hydrate thereof, unlike the existing method of recovering phosphoric acid in the form of disodium hydrogen phosphate (Korean Patent Publication No. 10-2025867). Therefore, when the phosphoric acid recovered according to the method of the present disclosure is reused in a fermentation process with a long fermentation time, for example, in the fermentation process of O-phosphoserine (OPS), etc., there are advantages that productivity is increased and the culture time is reduced. This means that the method may be very usefully applied even in the long fermentation process.

Experimental Example 2. Comparison of composition of recovered phosphoric acid waste liquid using O-phosphoserine fermentation waste liquid

The compositions (g/L) of the final recovered phosphoric acid waste liquids of Comparative Example 1 and Example 1 are shown in Table 2 below.

**[Table 2]**

| | Comparative Example 1 | Experimental Example 1 |
|---|---|---|
| Sodium concentration (g/L) | 92.4 | 68.6 |
| Ammonium concentration (g/L) | 13.1 | 44.1 |
| Phosphate concentration (g/L) | 61.2 | 59.2 |
| Sulfate concentration (g/L) | 139.3 | 153.1 |

As a result, as shown in Table 2, the composition (g/L) of the final recovered phosphoric acid waste liquid of Example 1 had 68.6 of sodium ion, 44.1 of ammonium ion, 59.2 of phosphate ion, and 153.1 of sulfate ion. In addition, the composition (g/L) of the final recovered phosphoric acid waste liquid of Comparative Example 1 had 92.4 of sodium ion, 13.1 of ammonium ion, 61.2 of phosphate ion, and 139.3 of sulfate ion.

In other words, the phosphoric acid waste liquid recovered by the method of Example 1 had the phosphate concentration similar to that of the phosphoric acid waste liquid recovered by the method of Comparative Example 1, but its ammonium content showed about 237% increase and its sodium content showed about 26% decrease.

These results indicate that the method of recovering phosphoric acid in the form of ammonium phosphate or a hydrate thereof from a fermentation medium and reusing the same according to the present disclosure may decrease the sodium ion content and may increase the ammonium ions in the recovered phosphoric acid waste liquid, and therefore, it may be usefully used as a high-value fertilizer.

### Experimental Example 3. Comparison of recovery rate of phosphoric acid using O-phosphoserine fermentation waste liquid

The phosphoric acid recovery rates of Comparative Example 1 and Example 1 are shown in Table 3 below.

**[Table 3]**

| | Comparative Example 1 | Experimental Example 1 |
|---|---|---|
| Initial phosphoric acid (g) | 85.0 | 85.0 |
| Phosphate (g) | 126.0 | 134.0 |
| Recovered phosphoric acid (g) | 54.0 | 57.0 |
| Phosphoric acid recovery rate (%) | 63.5 | 67.0 |

In Table 3, the initial phosphoric acid (g) means the weight of phosphoric acid in the process solution before concentration, and phosphate (g) means the weight of the recovered complex phosphate. The weight of only phosphoric acid in the recovered complex phosphate was expressed as the recovered phosphoric acid (g), and the phosphoric acid recovery rate (%) was calculated as a ratio of the recovered phosphoric acid (g) to the initial phosphoric acid (g).

As a result, as shown in Table 3, with regard to Example 1, the initial phosphoric acid was 85.0 g, the phosphate was 134.0 g, and the recovered phosphoric acid was 57.0 g. The total phosphoric acid recovery rate was 67.0 wt%. With regard to Comparative Example 1, the initial phosphoric acid was 85.0 g, the phosphate was 126.0 g, and the recovered phosphoric acid was 54.0 g. The total phosphoric acid recovery rate was 63.5 wt%.

In other words, when phosphoric acid was recovered by the method of Example 1, the phosphoric acid recovery rate showed about 3.5% increase, as compared to that of the method of Comparative Example 1.

These results indicate that since the method of recovering phosphoric acid in the form of ammonium phosphate or a hydrate thereof from the fermentation medium and reusing the same according to the present disclosure uses ammonia gas or ammonia solution in the pH-adjusting step, the phosphoric acid recovery rate is increased, as compared to the existing method (Korean Patent Publication No. 10-2025867) of recovering phosphoric acid, in which the phosphoric acid recovery rate is reduced due to dilution by using a sodium hydroxide solution in the pH-adjusting step.

This confirmed that the method of recovering phosphoric acid in the form of ammonium phosphate or a hydrate thereof from the fermentation medium and reusing the same according to the present disclosure has the effect of increasing productivity, i.e., the effect of increasing the phosphoric acid recovery rate from 63.5% to 67.0% and the effect of decreasing sodium ions, which are impurities in the phosphoric acid waste liquid, through the increased content of ammonium ions and the decreased content of sodium ions in the recovered phosphoric acid, as compared to the method of recovering phosphoric acid in the form of sodium hydroxide and reusing the same, and accordingly, the method of the present disclosure may be very usefully applied in fermentation processes.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of recovering phosphoric acid from a fermentation broth or a fermentation waste liquid, the method comprising the steps of:
(a) concentrating a phosphoric acid-containing fermentation broth or fermentation waste liquid;
(b) adjusting pH of the concentrated solution to 8 to 11 by adding ammonia gas or an ammonia solution to the concentrated solution; and
(c) separating ammonium phosphate or a hydrate thereof from the pH-adjusted concentrated solution.

2. A method of reusing phosphoric acid from a fermentation broth or fermentation waste liquid, the method comprising the steps of:
(a) concentrating a phosphoric acid-containing fermentation broth or fermentation waste liquid;
(b) adjusting pH of the concentrated solution to 8 to 11 by adding ammonia gas or an ammonia solution to the concentrated solution;
(c) separating ammonium phosphate or a hydrate thereof from the pH-adjusted concentrated solution; and
(d) adding the ammonium phosphate or the hydrate thereof to a medium.

3. The method of claims 1 or 2, wherein the ammonium phosphate is diammonium hydrogen phosphate.

4. The method of claims 1 or 2, wherein the fermentation broth comprises (i) O-phosphoserine, (ii) O-phosphohomoserine, or (iii) an amino acid or derivative thereof, prepared by adding O-phosphoserine or O-phosphohomoserine, a converting enzyme or a microorganism expressing the same, and a sulfide.

5. The method of claims 1 or 2, further comprising a step of cooling the pH-adjusted concentrated solution between step (b) and step (c), or in step (c).

6. The method of claims 1 or 2, further comprising a step of adding a crystal of ammonium phosphate or a hydrate thereof as a crystal nucleus between step (b) and step (c), or in step (c).

7. The method of claims 1 or 2, wherein the hydrate is diammonium hydrogen phosphate heptahydrate or diammonium hydrogen phosphate dodecahydrate.

8. The method of claims 1 or 2, further comprising a step of crystallizing the ammonium phosphate or the hydrate thereof of step (c) from a mother liquor.

9. The method of claim 8, wherein step (c) comprises (i) a step of concentrating a mother liquor, (ii) a step of adjusting pH of the mother liquor to 8 to 11, and (iii) a step of crystallizing ammonium phosphate or the hydrate thereof from the pH-adjusted mother liquor and separating the same.

10. The method of claim 9, further comprising a step of adding a crystal of the ammonium phosphate or the hydrate thereof as a crystal nucleus to the mother liquor between step (ii) and step (iii) or in step (iii).

11. The method of claims 1 or 2, further comprising a step of adjusting pH of the fermentation broth or the fermentation waste liquid to 8 to 11, before step (a).

12. The method of claims 1 or 2, further comprising a step of adding an alcohol in step (b); or between step (b) and step (c).

13. The method of claim 12, wherein the alcohol is methanol, ethanol, propanol, butanol, or a combination thereof.
